(19) Europäisches Patentamt
European Patent Office
Office européen des brevets

(11) Numéro de publication: **0 166 650**
**B1**

(12) # FASCICULE DE BREVET EUROPÉEN

(45) Date de publication du fascicule du brevet: **16.03.88**

(21) Numéro de dépôt: **85401168.1**

(22) Date de dépôt: **13.06.85**

(51) Int. Cl.⁴: **C 07 C 67/39**, C 07 C 69/65, C 07 C 79/46, C 07 C 103/76, C 07 D 277/18, C 07 C 69/734, C 07 C 102/08 // C07C121/70, C07D303/48

(54) **Procédé d'ouverture du cycle des gem dicyanoépoxydes et nouveaux composés obtenus par ce procédé.**

(30) Priorité: **15.06.84 FR 8409434**

(43) Date de publication de la demande: **02.01.86 Bulletin 86/1**

(45) Mention de la délivrance du brevet: **16.03.88 Bulletin 88/11**

(84) Etats contractants désignés: **AT BE CH DE FR GB IT LI LU NL SE**

(56) Documents cité: **Néant**

(73) Titulaire: **CENTRE NATIONAL DE LA RECHERCHE SCIENTIFIQUE (CNRS), 15, Quai Anatole France, F-75007 Paris (FR)**

(72) Inventeur: **Robert, Albert, 15, rue Courteline, F-35000 Rennes (FR)**
Inventeur: **Jaguelin, Sylvie, 7, rue de Douves, F-35600 Redon (FR)**
Inventeur: **Guinamant, Jean- Luc, Karret Bolazec, F-29216 Plougonven (FR)**

(74) Mandataire: **Jolly, Jean- Pierre, Cabinet BROT et JOLLY 83, rue d'Amsterdam, F-75008 Paris (FR)**

EP 0 166 650 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé d'ouverture du cycle des gem dicyanoépoxydes. L'invention concerne également, dans l'une de ses formes de mise en oeuvre, la préparation de composés arylacétiques, arylpropioniques et hétérocycliques à partir de gem dicyanoépoxydes. L'invention a enfin pour objet certains composés nouveaux obtenus en mettant en oeuvre ces procédés.

On sait que la réaction des époxydes avec des hydracides provoque l'ouverture du cycle époxyde et conduit à des chlorhydrines généralement stables.

L'ouverture des cycles épocydes des gem dicyanoépoxydes par des dérivés thiocarbonylés a également fait l'objet de diverses études ("Obtention d'intermédiaires tétrahédriques au cours de la réaction des gem dicyanoépoxydes avec les thioamides substitués. Evolution de ces intermédiaires en thiazoles mésoioniques", M. BAUDY et A. ROBERT, Tetrahedron Letters, Vol. 21, p. 2517-2520, 1980 ; voir également "A general synthesis of ring-fused mesoionic thiazolines from 2,2 -dicyanooxyranes under neutral conditions", M. BAUDY-FLOC'H, A. ROBERT, Synthesis, N° 12, December 1981, p. 981-984).

En poursuivant leur étude de l'ouverture du cycle des gem dicyano époxydes, les inventeurs ont établi que ces composés sont susceptibles de réagir avec des composés mono- ou binucléophiles en présence d'un hydracide, pour conduire à diverses familles de composés, notamment des composés arylacétiques ou arylpropioniques, lorsque le réactif est mono-nucléophile, et à des composés hétérocycliques, notamment de la famille des thiazoles et des imidazoles, lorsque le réactif est bi-nucléophile.

Un but de l'invention est donc de proposer un procédé d'ouverture du cycle des gem dicyanoépoxydes.

Un autre but de l'invention est de proposer de nouveaux procédés de synthèse de dérivés des acides arylacétiques et arylpropioniques et de composés hétérocycliques, notamment de la famille des thiazoles et des imidazoles.

Enfin, un autre but de l'invention est l'obtention de composés nouveaux des familles mentionnées ci-dessus.

A cet effet, l'invention a pour objet un procédé d'ouverture du cycle des gem dicyanoépoxydes, caractérisé en ce que l'on fait réagir le gem dicyano époxyde avec un composé mononucléophile en présence d'un acide choisi dans le groupe comprenant les hydracides et les acides faiblement nucléophiles.

Le procédé conforme à l'invention consiste donc à faire réagir en une étape, de façon sélective, deux réactifs nucléophiles différents - l'hydracide et un second nucléophile - sur des époxydes substitués par deux groupes partants cyano. En effet, contrairement à l'ouverture classique des époxydes par les hydracides, qui conduit à des chlorhydrines stables, les inventeurs ont établi, mais sans que la validité et la portée de la présente invention soient liées à cette théorie, que les gem dicyano-époxydes réagissent avec les hydracides pour donner des cyanhydrines instables, qui évoluent instantanément en des intermédiaires particulièrement intéressants, puisque substitués par un groupe cyanoformyle, qui, on le sait, sont des composés très réactifs. Le second nucléophile présent dans le milieu réagit immédiatement avec cet intermédiaire pour conduire avec de très bons rendements à un grand nombre de composés, dont certains sont nouveaux.

Si l'on désigne par H X l'hydracide et par Nu H le second réactif nucléophile, on a donc le schéma réactionnel suivant, dans le cas où Nu H est un réactif mononucléophile:

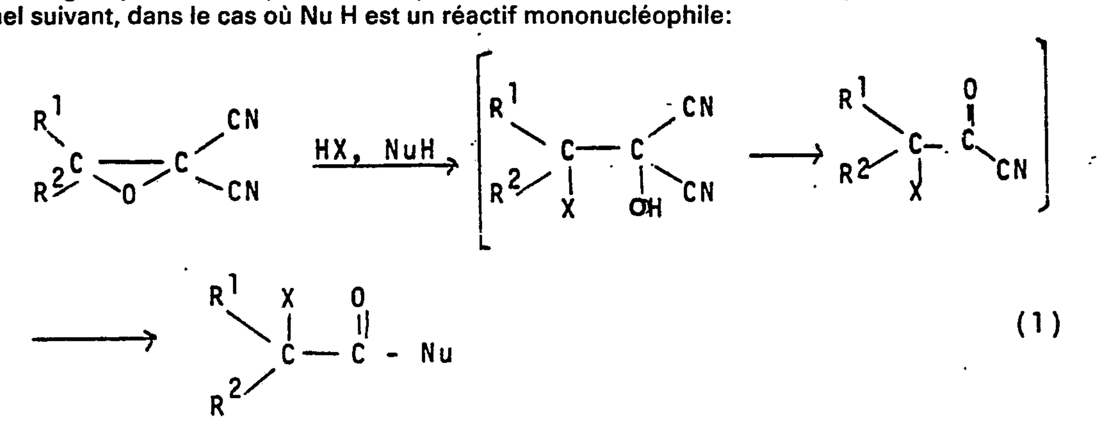


(1)

Dans cette réaction, les deux nucléophiles sont X et Nu, et X peut être un ion halogénure (Cl, Br, F) et Nu un autre nucléophile tel qu'un alcool ou une amine.

On remarquera que la gem disubstitution par des groupements cyano entraîne une ouverture univoque de l'époxyde, X substituant toujours et exclusivement le carbone portant les groupes $R^1$ et $R^2$. De plus, le second nucléophile présent dans le milieu (alcool ou amine) ne participe pas à l'ouverture du cycle époxyde, mais réagit toujours avec l'intermédiaire portant le groupe cyanoformyle.

Cette double sélectivité est à l'origine des rendements quasi quantitatifs en dérivés halogénés

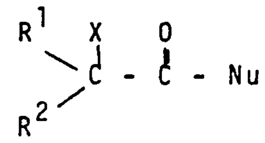

Dans le cas où Nu H est un réactif mononucléophile, la réaction (1) ci-dessus se ramène donc à l'addition sélective de deux nucléophiles (X et Nu) sur les deux carbones du cycle, tandis que les deux ions cyanures sont éliminés.

Les conditions de mise en oeuvre du procédé conforme à l'invention (température, pression, pH, quantités relatives de réactifs) n'ont pas de caractère critique.

Parmi les nombreux composés susceptibles d'être préparés par la réaction (1), les inventeurs ont notamment étudié les dérivés des acides arylacétiques et arylpropioniques.

L'invention a donc également pour objet un procédé de préparation des acides arylacétiques et arylpropioniques et de leurs dérivés consistant à faire réagir, en présence d'un hydracide choisi dans le groupe comprenant l'acide chlorhydrique, l'acide bromhydrique et l'acide fluorhydrique, un gem dicyanoépoxyde, dont un atome de carbone porte un groupe aryle, et un second réactif mononucléophile choisi dans le groupe comprenant l'eau, les alcools et les amines pour conduire aux esters α-halogénés, aux acides arylacétiques ou arylpropioniques α-halogénés et aux amides arylacétiques ou arylpropioniques α-halogénés correspondants.

Cette réaction s'écrit:

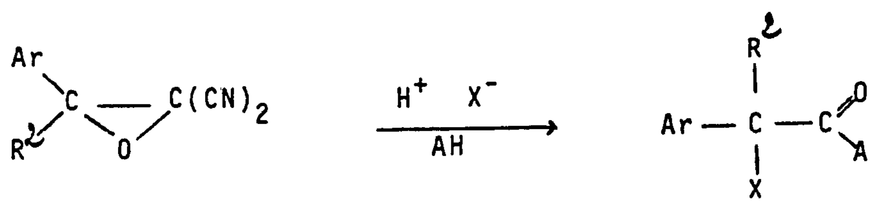


avec X = Cl, Br ou F et A = OH, OR, $NHR^1$
ou encore X = A = OR.

Lorsque l'hydracide est l'acide iodhydrique en solution aqueuse, sa réaction avec le gem dicyanoépoxyde (avec R = H) conduit directement à l'acide arylacétique non halogéné. Le dérivé halogéné intermédiaire est directement réduit par HI présent dans le milieu, suivant le schéma réactionnel suivant :

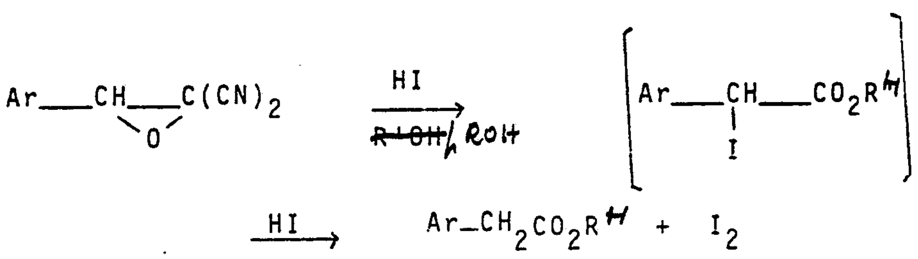


L'invention a donc aussi pour objet un procédé de préparation d'un acide arylacétique, caractérisé en ce que l'on fait réagir avec un alcool un gem dicyanoépoxyde dont un carbone porte un groupe aryle et un atome d'hydrogène, en présence d'acide iodhydrique.

Bien que les alcools ne soient pas assez acides pour ouvrir le cycle des époxydes, l'hydracide HX et le second mononucléophile Nu H de la réaction (1) ci-dessus peuvent être un seul et même composé (X = Nu = OR), à condition d'opérer en présence d'un acide peu nucléophile comme l'acide paratoluène sulfonique.

L'invention a donc également pour objet un procédé de préparation d'esters α-alcoxy - arylacétiques, caractérisé en ce que l'on fait réagir avec un alcool un gem dicyanoépoxyde dont un atome de carbone porte un groupe aryle et un atome d'hydrogène, en présence d'un acide faiblement nucléophile, par exemple de l'acide paratoluène sulfonique.

Cette réaction s'écrit:

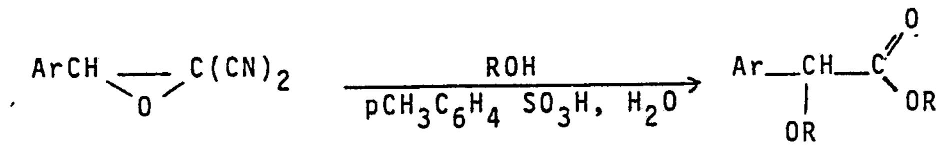


On va maintenant décrire en détail différentes formes de mise en oeuvre de l'invention et des exemples d'application des procédés mentionnés ci-dessus. Il est entendu que cette description ne vise qu'à illustrer l'invention et qu'elle n'a pas de caractère limitatif.

On se réfèrera d'abord à l'application du procédé conforme à l'invention à la synthèse des acides arylacétiques ou arylpropioniques et de leurs dérivés.

Ainsi qu'il a été indiqué ci-dessus, le schéma réactionnel du procédé, dans cette application, est le suivant :

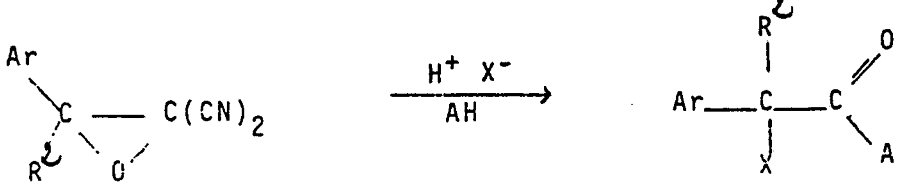


(I) (II)

où Ar désigne un groupe aryle et $R^2$ un autre groupe, X = Cl, Br ou F et A = OH, OR, $NHR^1$, ou encore X = A = OR.

Les gem di-cyano époxydes de départ peuvent être facilement obtenus par les réactions successives suivantes, qui sont bien connues dans la technique:

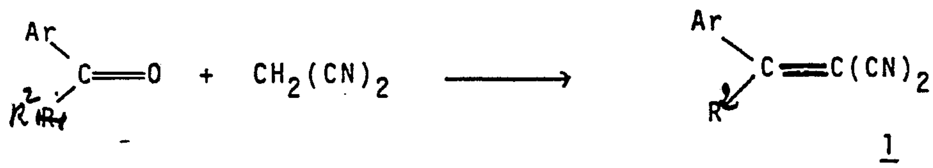

1

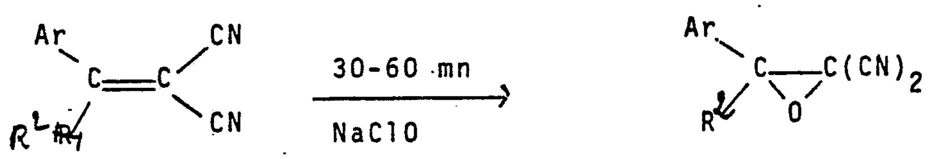


1°- Synthèse des esters α-halogénés (II)

Les esters α-halogénés II (avec X = Cl ou Br) sont obtenus avec de bons rendements par simple réaction des époxydes I avec un alcool, en présence d'une solution aqueuse d'un hydracide en quantité stoechiométrique. La réaction est totale après 3 h de reflux. Les esters α-fluorés II (X = F) sont préparés par réaction de l'acide fluorhydrique en solution dans de la pyridine, avec les époxydes I. Ceux-ci sont obtenus de façon quasi-quantitative par réaction d'eau de Javel avec les composés éthyléniques correspondants, qui résultent eux-mêmes de la condensation d'un aldéhyde ou d'une cétone avec le malononitrile (rendement quasi-quantitatif).

L'hydracide peut être HCl ou HBr en solution aqueuse ou HF en solution dans de la pyridine. Il est utilisé en léger excès par rapport à la stoechiométrie.

**EXEMPLE 1**

La synthèse de l'ester II avec Ar = $pClC_6H_4$ ; R=$(CH_3)_2CH$ ; X = Br, est décrite à titre d'exemple.

1a - Synthèse du composé éthylénique 1

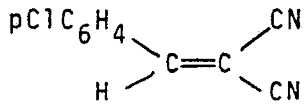

35 g (0,25 mole) de p-chlorobenzaldéhyde et 16,5 g (0,25 mole) de malononitrile sont mis en solution dans 120 $cm^3$ de dioxanne. On ajoute lentement 2 $cm^3$ de pipéridine et le mélange est agité à la température-ambiante pendant 30 mn. Le composé éthylénique précipite par addition de 300 $cm^3$ d'eau. Ce précipité est essoré, lavé à l'eau, puis séché (F = 160 °C, Rdt 96 %). Ce composé est suffisamment pur pour être directement utilisé dans l'étape ultérieure.

1b - <u>Synthèse de l'époxyde I</u>

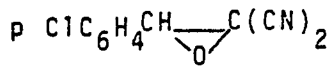

20 g (0,10 mole) du composé éthylénique 1 sont mis en solution dans 100 $cm^3$ d'acétonitrile. Le pH de la solution est ajusté (au papier pH) à 5-6 par addition d'acide sulfurique 2N. On ajoute 200 $cm^3$ d'hypochlorite de sodium 2,5 N, par fractions de 5 $cm^3$ à la température ambiante et sous agitation vigoureuse, tout en maintenant le pH aux environs de 5-6 par addition de $H_2SO_4$ 2N(20 $cm^3$ de $H_2SO_4$ 2N au total). On maintient l'agitation durant 30 mn puis on dilue par un litre d'eau. L'époxyde qui précipite est essoré et lavé à l'eau plusieurs fois avant d'être séché (F = 128-9 °C, Rdt 98 %). L'époxyde ainsi obtenu est utilisé sans autre purification dans la réaction suivante.

1c - <u>Synthèse de l'ester II</u>

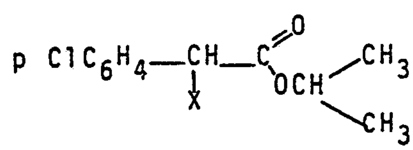

1 g d'époxyde ($5.10^{-2}$ mole) est mis en solution dans 30 $cm^3$ d'isopropanol. On ajoute 1 $cm^3$ d'acide bromhydrique à 48 % et on porte à reflux durant 3 heures. Après évaporation de l'isopropanol, on reprend par de l'éther (100 $cm^3$) et on lave l'éther par de l'eau, puis on sèche la solution éthérée sur sulfate de sodium. L'huile obtenue après évaporation de l'éther correspond à l'ester II pratiquement pur (Rdt 85 %). L'ester II est rectifié à l'aide d'un four tubulaire de Buchi (température du four 110° C, p = $1,5.10^{-2}$ mbar).

Le tableau ci-après rassemble les caractéristiques d'autres esters II chlorés ou bromés, préparés selon le même mode opératoire. Dans ce tableau, comme dans les tableaux qui suivront, les composés préparés sont caractérisés par leurs spectres infrarouge I R, de résonance magnétique nucléaire RMN et de masse. Ils présentent une analyse centésimale correcte. Dans les différents tableaux, les composés désignés par le signe * sont nouveaux, à la connaissance des inventeurs, et, comme tels, constituent un objet de l'invention.

L'ester fluoré pour lequel Ar = $pCH_3C_6H_4$, $R^2$ = H et R = $CH_3$ a été préparé selon le mode opératoire suivant:

1 g d'époxyde I (Ar = $pMeC_6H_4$, R = H) est mis en solution dans 10 $cm^3$ de dichlorométhane. On ajoute ensuite 20 $cm^3$ d'une solution HF (70 g)/pyridine (30 g) et on porte au réfrigérateur (2° C) pendant 3 jours. Après addition de 50 $cm^3$ de méthanol, le milieu réactionnel est neutralisé par une solution d'ammoniaque à 28 %. Après extraction à l'éther et séchage, puis évaporation de l'éther, on isole l'ester α-fluoré, Eb = 70°C $1.10^{-2}$ mbar, Rdt 80 %.

TABLEAU I

Esters α-halogénés I (R=H) 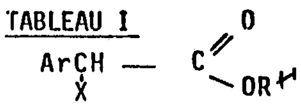

| Ar | $R^1$ | X | Rdt %* | Eb°C P mbar | Formule | Masse th / tr | IR (film liq.) νCO ($cm^{-1}$) | RMN $CDCl_3$/TMS | δ (ppm) |
|---|---|---|---|---|---|---|---|---|---|
| $C_6H_5$ | $CH_3$ | Br | 90 | 95 / $2\ 10^{-2}$ | $C_9H_9BrO_2$ | 227,9785 / 227,978 | 1745 | 3,60(s,3H) 5,30(s,1H) | 7,12-7,62(m,5H) |
| $pClC_6H_4$ | $CH_3$ | Br | 85 | 110 / $3\ 10^{-2}$ | $C_9H_8BrClO_2$ | 261,9396 / 261,941 | 1746 | 3,77(s,3H) 5,30(s,1H) | 7,25-7,55(m,4H) |
| $pClC_6H_4$ | $C_2H_5$ | Br | 88 | 125 / $5\ 10^{-2}$ | $C_{10}H_{10}BrClO_2$ | 275,9552 / 275,955 | 1740 | 1,25(t,3H) 4,20(q,2H)5,35(s,1H) | 7,20-7,40(m,4H) |
| $pClC_6H_4$ | $C_2H_5$ | Cl | 90 | 100 / $2\ 10^{-2}$ | $C_{10}H_{10}Cl_2O_2$ | 232,0057 / 232,004 | 1748 | 1,22(t,3H)4,20(q,2H) 5,32(s,1H) | 7,25-7,50(m,4H) |
| $pClC_6H_4$ | $(CH_3)_2CH$ | Br | 85 | 110 / $1,5\ 10^{-2}$ | $C_{11}H_{12}BrClO_2$ | 291,9688 / 291,969 | 1735 | 1,20(d,3H)1,27(d,3H) 5,05(m,1H)5,27(s,1H) | 7,22-7,55(m,4H) |
| $pNO_2C_6H_4$ | $C_2H_5$ | Br | 95 | 145 / $4\ 10^{-2}$ | $C_{10}H_{10}BrNO_4$ | 286,9793 / 286,981 | 1740 | 1,30(t,3H) 4,27(q,2H)5,47(s,1H) | 7,70-8,25(m,4H) |
| $pNO_2C_6H_4$ | $C_2H_5$ | Cl | 90 | 140 / $2\ 10^{-2}$ | $C_{10}H_{10}ClNO_4$ | 243,0298 / 243,029 | 1742 | 1,20(t,3H) 4,22(q,2H)5,40(s,1H) | 7,68-8,30(m,4H) |

De façon analogue, le Tableau II ci-après rassemble les caractéristiques de différents α-halogène esters propioniques et des esters propioniques qui en dérivent par réduction par le zinc en milieu acide acétique-eau.

TABLEAU II

Esters propioniques 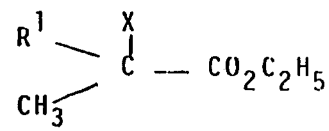

| $R^1$ | X | Rdt % | RMN ($CDCl_3$) δ ppm | IR film liq. $cm^{-1}$ |
|---|---|---|---|---|
| $C_6H_5$ | Cl | 90 | 1,20 (t, 3H), 2,12 (s, 3H) 4,30 (q, 2H) 7,10 - 7,70 (m, 5H) | 1726 |
| $mBrC_6H_4$ | Cl | 70 | 1,27 (t, 3H), 2,12 (s, 3H) 4,25 (q, 2H) 7,00 - 7,90 (m, 4H) | 1730 |
| $C_6H_5$-$CH_2$ | Br | 80 | 1,30 (t, 3H), 1,85 (s, 3H) 3,50 (centre syst AB, 2H)4,25(q, 2H) 7,25 (s, 5H) | 1728 |
| $CO_2C_2H_5$ | Cl | 80 | 1,32 (t, 6H), 1,94 (s,3H) 4,30(q, 4H) | 1741 |
| $C_6H_5$ | H | 90 | 1,22 (t, 3H), 1,52 (d, 3H) 3,72 (q, 1H) 4,12 (q, 2H) 7,10 - 7,60 (m,5H) | 1728 |
| $mBrC_6H_4$ | H | 70 | 1,23 (t, 3H) 1,52 (d, 3H) 3,70 (q, 1H) 4,15 (q, 2H) 7,00 - 7,60 (m,4H) | 1728 |
| $CO_2C_2H_5$ | H | 80 | 1,30 (, 6H), 1;45 (d, 3H) 3,45 (q, 1H) 4,42 (q, 4H) | 1730 |

Suite TABLEAU 1

Esters α-halogénés I (R=H) $ArCH(X)—C(=O)OR^1$

| Ar | $R^1$ | X | Rdt % (1) | Eb°C P mbar | Formule | Masse th / tr | IR(film liq.) $\nu_{CO}(cm^{-1})$ | RMN $CDCl_3$/TMS | δ (ppm) |
|---|---|---|---|---|---|---|---|---|---|
| $mNO_2C_6H_4$ | $(CH_3)_2CH$ | Br | 85 | 135<br>$2\ 10^{-2}$ | $C_{11}H_{12}NO_4Br$ | 300,9949<br>300,994 | 1730 | 1,30(d,3H) 1,37(d,3H)<br>5,10(m,1H) 5,40(s,1H) | 7,45-8,50(m,4H) |
| $pCH_3C_6H_4$ | $C_2H_5$ | Br | 86 | 105<br>$3\ 10^{-2}$ | $C_{11}H_{13}BrO_2$ | 256,0098<br>256,008 | 1743 | 1,22(t,3H) 2,30(s,3H)<br>4,17(q,2H) 5,30(s,1H) | 7,05-7,47(m,4H) |
| $pCH_3C_6H_4$ | $CH_3$ | F | 80 | 70<br>$2\ 10^{-2}$ | $C_{10}H_{11}FO_2$ | - | 1757 | 2,35(s,3H) 3,72(s,3H)<br>5,72(d,1H<br>JHF = 47 Hz) | 7,00-7,50(m,4H) |

(1) Rendements calculés à partir des époxydes I.

A titre d'exemple de la préparation des esters propioniques, on décrit maintenant la synthèse de l'ester propionique de formule

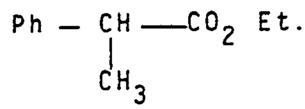

**EXEMPLE 2**

1 g d'ester α-halogéné

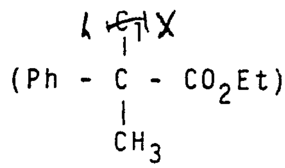

est mis en solution dans 20 $cm^3$ d'acide acétique et 10 $cm^3$ d'eau. On ajoute 3 g de zinc en poudre et on porte à reflux pendant 4 heures. On étend la solution à 250 $cm^3$ par de l'eau et on extrait par de l'éther (4 x 50 ml). La phase éthérée est lavée avec une solution de soude N puis avec de l'eau. L'éther est séché sur sulfate de sodium, puis évaporé. L'huile obtenue ne présente pas d'impureté à l'échelle de la RMN. Le rendement de la réaction est quantitatif et le composé est caractérisé par ses spectres IR, RMN (Tableau II).

Le Tableau II montre que le procédé selon l'invention permet d'accéder à l' α-chloro α-méthyl malonate d'éthyle (R = $CO_2Et$, X = Cl) ou à son dérivé réduit (R = $CO_2Et$, X = H) et constitue par suite une voie d'accès à des dérivés monoalcoylés du malonate d'éthyle (on sait que de tels composés ne peuvent pas être préparés de façon sélective par alcoylation directe du malonate d'éthyle). Les inventeurs ont également préparé dans de bonnes conditions et de façon analogue le phénylmalonate d'éthyle.

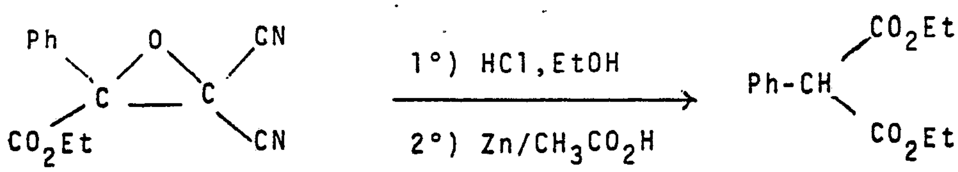


2° - <u>Synthèse des esters arylacétiques</u> Ar--$CH_2CO_2$ R'

La synthèse de l'ester Ph-$CH_2CO_2C_2H_2$ est donnée à titre d'exemple.

**EXEMPLE 3**

1 g d'époxyde I (Ar = Ph) préparé selon le mode opératoire décrit plus haut (1a) (en remplaçant l'aldéhyde p-chlorobenzoïque par de l'aldéhyde benzoïque) est mis en solution dans 30 $cm^3$ d'éthanol. On ajoute 1 $cm^3$ d'acide iodhydrique à 67 % et on porte à reflux deux heures. Après évaporation de l'éthanol, on reprend l'huile résiduelle par de l'éther et on lave l'éther avec une solution de thiosulfate jusqu'à décoloration. L'éther est séché sur sulfate de sodium anhydre puis évaporé. Un spectre de RMN de l'huile obtenue ne permet pas de déceler d'impureté. L'ester ainsi obtenu (Rdt 70 %, non optimisé) présente les mêmes spectres IR et RMN qu'un échantillon de phényl acétate d'éthyle du commerce.

3° - <u>Synthèse des acides arylacétiques α-halogénés</u>

Les acides arylacétiques α-halogénés III sont obtenus de façon analogue aux esters II correspondants ($R^2$ = H), en remplaçant l'alcool par du tétrahydrofurane THF.

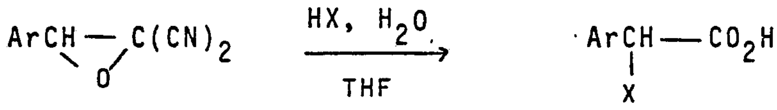


III

La synthèse de l'acide

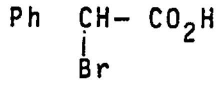

est décrite ci-après à titre d'exemple.

**EXEMPLE 4**

1 g d'époxyde I (Ar = Ph) en solution dans un mélange THF (30 $cm^3$), HBr à 48 % (1 $cm^3$) est porté à reflux pendant trois heures. Le THF est ensuite évaporé sous pression réduite et le milieu est extrait par de l'éther. L'acide est extrait de la phase éthérée par un lavage avec une solution de NaOH N (20 $cm^3$). La phase aqueuse est ensuite acidifiée par HCl 1/3 et l'acide est extrait par de l'éther. L'éther est séché sur sulfate de sodium, puis évaporé, et l'acide obtenu (F = 82°C; Rdt 70 %) est identifié en comparant ses caractéristiques (IR et RMN) à celles d'un échantillon du commerce.

4° - <u>Synthèse des amides arylacétiques α-halogénés</u>

Les amides arylacétiques α-halogénés IV sont obtenus à partir de la réaction des époxydes I en solution dans de l'acétonitrile en présence de la quantité stoechiométrique d'un hydracide en solution aqueuse et d'une amine. La réaction est totale après 16 h à la température ambiante.

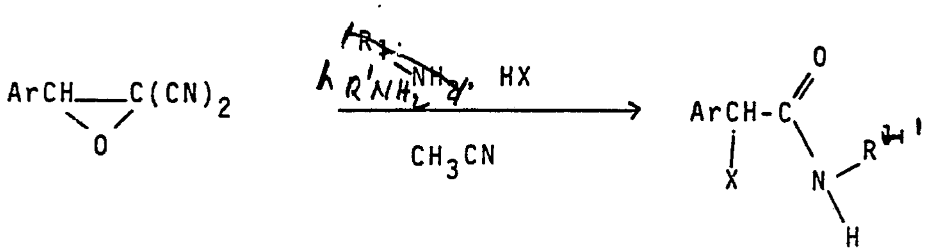


IV

Le Tableau III ci-après rassemble les caractéristiques de divers amides arylacétiques α-halogénés obtenus par le procédé de l'invention. L'exemple 5 qui suit illustre la préparation de certains de ces amides.

## TABLEAU III

Amides α-halogénés

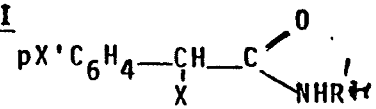

| X' | X | R'/R' | Rdt % | $\theta_f$ °C | Formule | Masse M+ Calc / Tr | RMN δppm $CDCl_3$ + $CF_3CO_2H$) | IR $cm^{-1}$ (Nujol) |
|---|---|---|---|---|---|---|---|---|
| Cl | Br | $CH_3CH_2$ | 90 | 120 | $C_{10}H_{11}NOBrCl$ | 274,9712<br>274,971 | 1,20 : t,3H)<br>3,30 : (m, 2H)<br>5,40 : (s, 1H)<br>7,35 : (m, 4H) | ~NH : 3270<br>~CO : 1646 |
| Cl | Br | $CH_3$ | 90 | 114 | $C_9H_9NOBrCl$ | 260,9556<br>260,952 | 2,90 : (d, 3H)<br>5,40 : (s, 1H)<br>7,35 : (m, 4H) | ~NH : 3277<br>~CO : 1669 |
| H | Cl | $CH_3$ | 80 | 76 | $C_9H_{10}NOCl$ | 183,0450<br>183,044 | 2,80 : (t, 3H)<br>5,40 : (s, 1H)<br>7,35 : (m, 5H) | ~NH : 3268<br>~CO : 1655 |
| Cl | Cl | $C_6H_5$ | 96 | 122 | $C_{14}H_{11}NOCl_2$ | 279,0217<br>279,021 | 5,57 : (s, 1H)<br>7;35 : (m, 9H) | ~NH : 3265<br>~CO : 1669 |
| Cl | Cl | $CH_3CH_2$ | 76 | 106 | $C_{10}H_{11}NO^{35}Cl^{37}Cl$ | 233,0188<br>233,021 | 1,20 : (t, 3H)<br>3,35 : (m, 2H)<br>5,35 : (s, 1H)<br>7,35 : (s, 4H) | ~NH : 3270<br>~CO : 1665 |
| Me | F | H | 40 (non optimisé) | 133 | $C_9H_{10}FNO$ | 167,0746<br>167,074 | 2,40 (d, 3H; $J_{HF}$=2Hz)<br>5,77 (d, 1H; $J_{HF}$=47Hz)<br>7,15 à 7,35 (m, 4H) | ~NH : 3380<br>3150<br>~CO: 1656 |

Suite TABLEAU III

Amides α-halogénés 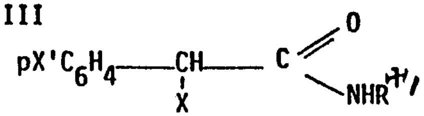

| X' | X | R' | Rdt % | $\theta_f$ °C | Formule | Masse $M^+$ Calc / Tr | RMN δppm ($CDCl_3$ + $CF_3CO_2H$) | IR $cm^{-1}$ (Nujol) |
|---|---|---|---|---|---|---|---|---|
| $CH_3$ | Cl | $CH_2CH_2OH$ | 90 | 102 | $C_{11}H_{14}NO_2Cl$ * | 227,0713<br>227,072 | 2,40 (s, 3H)<br>3,45 (t, 2H) 3,65(t,2H)<br>5,35 (s, 1H) 7,20(m,4H) | ~NH : 3305<br>~CO : 1640 |
| Cl | Cl | $CH_2CH_2Cl$ | 98 | 82 | $C_{10}H_{10}NOCl_3$ * | 264,9827<br>264,982 | 3,70 (d, 4H) 5,40(s,1H)<br>7,35 (s, 4H) | ~NH : 3270<br>~CO : 1646 |
| $NO_2$ | Cl | $CH_2CH_2Cl$ | 92 | 90 | $C_{10}H_9N_2O_3$ * | $(M-Cl-HCl)^+$<br>205,0613<br>205,060 | 3,70 (d, 4H)<br>5,50 (s, 1H)<br>7,90 (m, 4H) | ~NH : 3296<br>~CO : 1654 |
| H | Cl | $CH_2CH_2Cl$ | 96 | 84 | $C_{10}H_{11}NOCl_2$ * | 231,0217<br>231,021 | 3,60 (d, 4H)<br>5,40 (s, 1H)<br>7,40 (s, 5H) | ~NH : 3311<br>~CO : 1648 |
| Cl | Cl | $CH_2CH_2SCN$ | 70 | 106 | $C_{11}H_{10}N_2OSCl_2$ * | 287,9891<br>287,988 | 3,65 (d, 4H)<br>5,25 (s, 1H)<br>7,40 (s, 4H) | ~NH : 3275<br>~C ≡N : 2149<br>~CO : 1635 |

* composé nouveau

**EXEMPLE 5**

4a - Préparation des amides arylacétiques α-halogénés IV avec X = Cl ou X = Br.

La synthèse de l'amide

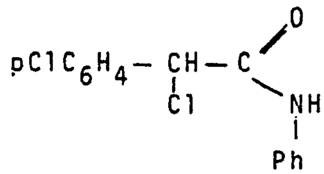

est décrite ci-après à titre d'exemple.

1 g d'époxyde est mis en solution dans 20 $cm^3$ d'acétonitrile 1ml de HCl 12 N et 0,46 g d'aniline sont ajoutés au milieu réactionnel. On laisse sous agitation à la température ambiante pendant 16 h puis on distille le solvant. L'huile résiduelle est reprise avec de l'éther et lavée à la soude N. L'évaporation de l'éther conduit à l'amide (F = 122°C, Rdt 96 %).

4b - Synthèse de l'α-fluoroamide IV avec X' = Me, X = F, R = H

1 g d'époxyde I (Ar = $pMeC_6H_4$, R = H) est mis en solution dans 10 $cm^3$ dé dichlorométhane. On ajoute ensuite 20 $cm^3$ d'une solution HF (70 g)/pyridine (30 g) et porte au réfrigérateur (2°C) pendant 3 jours. Le milieu réactionnel est neutralisé par de l'ammoniaque à 28 %. Après extraction avec du dichlorométhane et séchage, puis évaporation du solvant, l'amide fluoré précipite, F = 133 °C (éther-éther de pétrole), Rdt 40 % (non optimisé).

4c - Synthèse de l'amide α-chloré IV avec X = Cl, R = $CH_2CH_2SCN$.

Ce composé est préparé selon le schéma suivant :

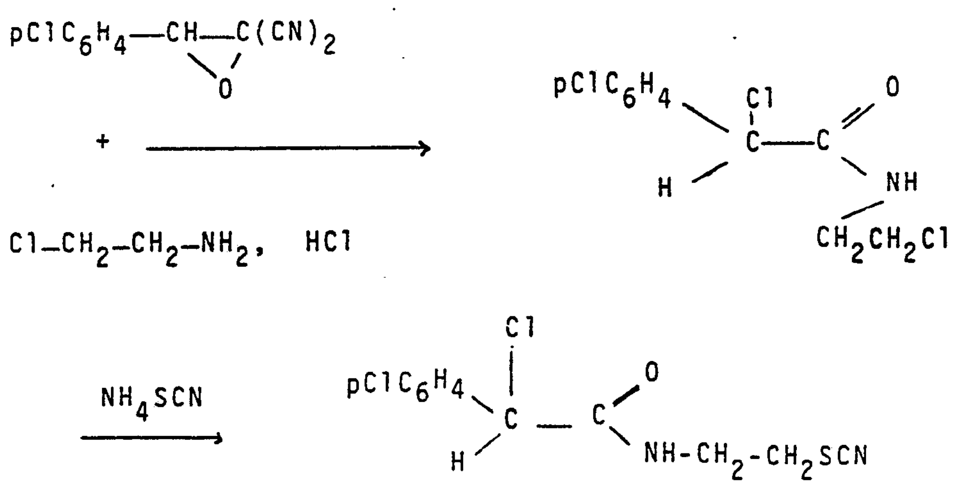


A 2 g d'amide halogéné IV (X = Cl, R' = $CH_2CH_2Cl$) (préparé selon le mode opératoire 4a décrit plus haut) en solution dans 30 $cm^3$ de toluène on ajoute 1,5 g de thiocyanate d'ammonium en solution dans 20 $cm^3$ d'eau. On ajoute 0,4 g de chlorure de benzyltriéthylammonium et on porte à reflux sous agitation pendant 24 h. La phase organique est décantée, lavée à l'eau, et séchée sur sulfate de sodium, puis évaporée. Le solide qui précipite est l'amide IV (R' = $CH_2CH_2SCN$) F = 106°C, Rdt 70 %.

L'amide ainsi obtenu semble particulièrement intéressant, car les inventeurs ont montré que ce composé peut être cyclisé en présence de triéthylamine pour donner l'oxo- 5 tétramisole.

5 - Synthèse des esters arylacétiques α-alcoylés.

Ainsi qu'il a été indiqué ci-dessus les esters α-alcoxyarylacétiques V peuvent être préparés selon le processus suivant:

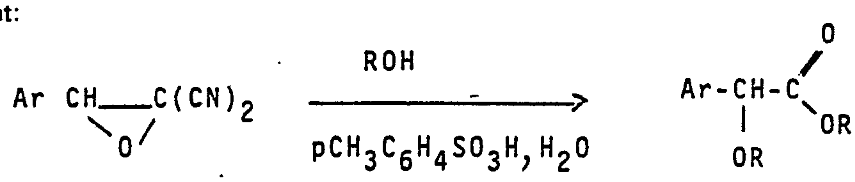


V

La synthèse de l'ester V avec Ar = p Cl $C_6H_4$ et R = Et va être décrite dans l'Exemple 6 ci-après:

**EXEMPLE 6**

2 g d'époxyde I (Ar = $pClC_6H_4$) et 3,8 g d'acide paratoluène sulfonique monohydraté sont portés à reflux pendant 48 h dans 30 $cm^3$ d'éthanol. Après évaporation du solvant, l'huile résiduelle est reprise par 50 $cm^3$ d'éther et lavée par 20 $cm^3$ de soude 2N et par de l'eau (4 fois 50 ml). Après séchage sur $Na_2SO_4$, l'éther est évaporé. L'ester

$$Ar-\underset{\displaystyle OEt}{\underset{|}{CH}}-CO_2Et$$

est obtenu avec 65 % de rendement, et aucune impureté ne peut être decelée dans les limites de la sensibilité de la RMN.

Le Tableau V ci-après rassemble les caractéristiques d'autres esters arylacétiques α-alcoylés préparés conformément à l'invention.

TABLEAU V

Esters arylacétiques α-alcoylés $Ar-\underset{\displaystyle OR}{\underset{|}{CH}}-CO_2R$ 7

| Référence | Ar | R | Rdt% | Formule | Masse | IR(film liq.) $\nu_{CO}$ $cm^{-1}$ | RMN $CDCl_3$/TMS | δ ppm |
|---|---|---|---|---|---|---|---|---|
| 29 | $C_6H_5$ | $C_2H_5$ | 60 | $C_{12}H_{16}O_3$ | 208,1099<br>208,110 | 1746 | 1,18 (t, 3H) 1,26(t, 3H)<br>3,25 à 3,80 (m, 2H)<br>4,15 (q, 2H)<br>4,86 (s, 1H) | 7,20 à 7,60 (m, 5H) |
| 25 | $C_6H_5$ | $CH_3$-CH- $CH_3$/ | 50 | $C_{14}H_{20}O_3$ | Observé<br>$M-CO_2CH(CH_3)_2^+$<br>149,0966<br>149,096 | 1750<br>1728 | 1,12 (d, 3H) 1,21(d,3H)<br>1,24 (d, 3H) 1,27(d,3H)<br>3,71 (septet, 1H)<br>4,94 (s, 1H)<br>5,04 (septet, 1H) | 7,15 à 7,70 (m, 5H) |
| 25 | $pNO_2C_6H_4$ | $C_2H_5$ | 75 | $C_{12}H_{15}NO_5$ | 253,0950<br>253,095 | 1745<br>1734 | 1,27 (t, 3H) 1,35 (t,3H)<br>3,67 (m, 2H) 4,22 (q,2H)<br>5,00 (s, 1H) | 7,60 à 8,40 (m, 4H) |
| 30 | $pClC_6H_4$ | $C_2H_5$ | 65 | $C_{12}H_{15}ClO_3$ | 242,0709<br>242,071 | 1743 | 1,25 (t, 3H) 1,30 (t,3H)<br>3,60 (m, 2H) 4,20 (q,2H)<br>4,85 (s, 1H) | 7,35 (m, 4H) |

**Revendications**

1. Procédé d'ouverture du cycle des gem dicyanoépoxydes, caractérisé en ce que l'on fait réagir le gem dicyanoépoxyde avec un composé mononucléophile en présence d'un acide choisi dans le groupe comprenant les hydracides et les acides faiblement nucléophiles.

2. Procédé selon la revendication 1, appliqué à la préparation des acides arylacétiques et arylpropioniques et de leurs dérivés, consistant à faire réagir, en présence d'un hydracide choisi dans le groupe comprenant l'acide chlorhydrique, l'acide bromhydrique et l'acide fluorhydrique, un gem dicyanoépoxyde, dont un atome de carbone porte un groupe aryl, et un second réactif mononucléophile choisi dans le groupe comprenant l'eau, les alcools, et les amines pour conduire aux esters α-halogénés, aux acides arylacétiques ou arylpropioniques α-halogénés et aux amides arylacétiques ou arylpropioniques α-halogénés correspondants.

3. Procédé selon la revendication 1, appliqué à la préparation d'un acide arylacétique, caractérisé en ce que l'on fait réagir avec un alcool un gem diayanoépoxyde dont un carbone porte un groupe aryle et un atome d'hydrogène, en présence d'acide iodhydrique.

4. Procédé selon la revendication 1, appliqué à la préparation d'esters α-alcoxy-arylacétiques, caractérisé en ce que l'on fait réagir avec un alcool un gem dicyanoépoxyde dont un atome de carbone porte un groupe aryle et un atome d'hydrogène, en présence d'un acide faiblement nucléophile, par exemple de l'acide paratoluène sulfonique.

5. Les amides α-halogénés de formule :

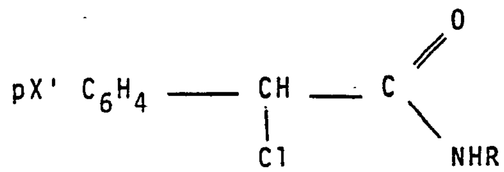

où X' et R' désignent respectivement un groupe méthyle et un groupe $CH_2CH_2OH$, un atome de chlore et un groupe $CH_2CH_2Cl$, un groupe $NO_2$ et un groupe $CH_2CH_2Cl$, un atome d'hydrogène et un groupe $CH_2CH_2Cl$, un atome de chlore et un groupe $CH_2CH_2SCN$.

**Patentansprüche**

1. Verfahren zum Öffnen des Ringes von gem-Dicyanoepoxiden, dadurch gekennzeichnet, daß das gem-Dicyanoepoxid mit einer mononukleophilen Verbindung in Gegenwart einer Säure, gewählt aus der Gruppe der Wasserstoffsäuren und der schwach nukleophilen Säuren, umgesetzt wird.

2. Verfahren nach Anspruch 1, zur Herstellung von Arylessigund Arylpropionsäuren sowie deren Derivate, dadurch gekennzeichnet, daß ein gem-Dicyanoepoxid, welches an einem Kohlenstoffatom eine Arylgruppe trägt, in Gegenwart einer Wasserstoffsäure, gewählt aus der Gruppe Chlor-, Brom- und Fluorwasserstoffsäure, mit einem zweiten reaktiven Mononukleophil, gewählt aus der Gruppe Wasser, Alkohole und Amine, zu α-halogenierten Estern, α-halogenierten Arylessig- oder Arylpropionsäuren und den entsprechenden α-halogenierten Arylacet- und Arylpropionamiden umgesetzt wird.

3. Verfahren nach Anspruch 1, zur Herstellung einer Arylessigsäure, dadurch gekennzeichnet, daß ein gem-Dicyanoepoxid, welches an einem Kohlenstoffatom eine Arylgruppe und ein Wasserstoffatom trägt, mit einem Alkohol in Gegenwart von Iodwasserstoffsäure umgesetzt wird.

4. Verfahren nach Anspruch 1, zur Herstellung von α-Alkoxyarylessigsäureestern, dadurch gekennzeichnet, daß ein gem-Dicyanoepoxid, welches an einem Kohlenstoffatom eine Arylgruppe und ein Wasserstoffatom trägt, mit einem Alkohol in Gegenwart einer schwach nukleophilen Säure, insbesondere Paratoluolsulfonsäure, umgesetzt wird.

5. Die α -halogenierten Amine der Formel

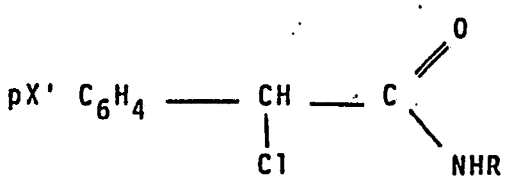

worin X' und R' jeweils eine Methylgruppe eine $CH_2CH_2OH$-Gruppe, ein Chloratom und eine $CH_2CH_2Cl$-Gruppe, eine $NO_2$-Gruppe und eine $CH_2CH_2Cl$-Gruppe, ein Wasserstoffatom und eine $CH_2CH_2Cl$-Gruppe, ein Chloratom und eine $CH_2CH_2SCN$-Gruppe bedeuten.

**Claims**

1. A process for opening the ring of gem dicyanoepoxides characterised by reacting the gem dicyanoepoxide with a mononucleophilic compound in the presence of an acid selected from the group comprising hydracides and acids which are weakly nucleophilic.

2. A process according to claim 1 applied to the preparation of arylacetic and arylpropionic acids and derivatives thereof, comprising reacting, in the presence of a hydracide selected from the group comprising hydrochloric acid, hydrobromic acid and hydrofluoric acid, a gem dicyanoepoxide in which a carbon atom carries an aryl group, and a second mononucleophilic reactant selected from the group comprising water, alcohols and amines to produce α-halogenated esters, α-halogenated arylacetic or arylpropionic acids and the corresponding α-halogenated arylacetic or arylpropionic amides.

3. A process according to claim 1 applied to the preparation of an arylacetic acid characterised by reacting with an alcohol a gem dicyanoepoxide in which a carbon carries an aryl group and a hydrogen atom, in the presence of hydriodic acid.

4. A process according to claim 1 applied to the preparation of α-alkoxy-arylacetic esters characterised by reacting with an alcohol a gem dicyanoepoxide in which a carbon atom carries an aryl group and a hydrogen atom in the presence of a weakly nucleophilic acid, for example paratoluene sulphonic acid.

5. α -Halogemated amides of the following formula:

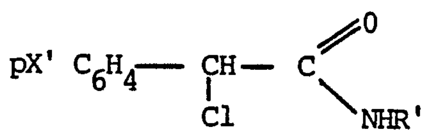

wherein X' and R' respectively denote a methyl group and a group $CH_2CH_2OH$, a chlorine atom and a group $CH_2CH_2Cl$, a group $NO_2$ and a group $CH_2CH_2Cl$, a hydrogen atom and a group $CH_2CH_2Cl$, a chlorine atom and a group $CH_2CH_2SCN$.